# EUROPÄISCHE PATENTSCHRIFT

(11) **EP 0 331 700 B1**
(45) Veröffentlichungstag und Bekanntmachung des Hinweises auf die Patenterteilung: **03.11.1993**
(21) Anmeldenummer: 88907651.9
(22) Anmeldetag: 25.08.1988
(51) Int. Cl.: A61F 2/28, A61L 27/00

(54) **VORSPANNBARES CHIRURGISCHES NETZWERK**
PRESTRESSABLE SURGICAL NETWORK
STRUCTURE RETICULEE PRECONTRAINTE A USAGE CHIRURGICAL

(30) Priorität: 27.08.1987 DE 3728686
(43) Veröffentlichungstag der Anmeldung: 13.09.1989
(73) Patentinhaber: DRAENERT, Klaus, Dr.med., D-81545 München (DE)
(72) Erfinder: DRAENERT, Klaus, Dr.med., D-81545 München (DE)
(74) Vertreter: VOSSIUS & PARTNER
(86) Internationale Anmeldenummer: EP8800761
(87) Internationale Veröffentlichungsnummer: WO8901766

(56) Entgegenhaltungen:
- EP-A- 0 006 414
- EP-A- 0 159 036
- EP-A- 0 212 087
- CH-A- 665 348
- DE-A- 3 637 314
- DE-A- 3 707 518
- DE-B- 2 404 214
- US-A- 4 089 071
- US-A- 4 373 217
- US-A- 4 495 664

## Beschreibung

Die Erfindung betrifft ein chirurgisches Trag- oder Netzwerk wie es im Oberbegriff des Anspruchs 1 angegeben ist. Ein solches Netzwerk ist aus der EP-A-0 159 036 bekannt.

Die Verankerung von Gelenkersatzkomponenten im Knochen erfolgt üblicherweise entweder mit Hilfe eines kaltpolymerisierenden Polymethylmetacrylat (PMMA)-Kunststoffes oder durch direkte Verblockung im Knochen ohne Knochenzement. Neuerdings wird auch versucht, durch zugverspannte Konstruktionen Implantatkomponenten mehr physiologisch zu verankern (A.H. Huggler: Die Druckscheibenprothese im siebten Jahr ihrer klinischen Anwendung, in Draenert, K. und Rütt A.(Hrsg); Histo-Morphologie des Bewegungsapparates 3, Art and Science, München, 1987).

Die Verankerung von künstlichen Gelenkersatzkomponenten im Knochen scheitert sehr oft daran, daS die Kräfte nicht gleichmäßig auf den Knochen übertragen werden können, was zur Folge hat, daß bei zementfreien Komponenten mit einem hohen Elastizitätsmodul eine Streßkonzentration (Spannungsspitze) in umschriebenen Bereichen, wo der größte Teil der Last aufgenommen wird, zur Kompaktisierung des Knochens führt, während die übrigen Knochenabscbnitte atrophieren, da sie nicht mehr beansprucht werden. Bei zementierten Komponenten kann der Zement sehr oft den hohen Lasten auf lange Dauer nicht Stand halten und bricht, was letztlich die Lockerung der Gelenkersatzkomponente zur Folge hat.

Von allen auftretenden Kräften haben die Scherkräfte die ungünstigste Auswirkung auf die Differenzierung und Entwicklung des pluripotenten Mesenchyms, des Keimgewebes, aus dem letztlich die knochenbildenden Zellen, die Osteoblasten, hervorgehen.

Aus der Architektur und Baustatik sind Konstruktionen bekannt, bei denen versucht wird, solche ungünstigen Scherkräfte durch eine Vorspannung abzufangen, nämlich durch Armierung der Betonkonstruktionen durch vorgespannte Metallnetze, die die Zugspannungen aufnehmen (Spannbeton). Auch durch freie zugverspannte Tragwerke, beispielsweise in Form leichter Flächentragwerke, lassen sich Zugkräfte in Druckkräfte umwandeln (Frei-Otto: Natürliche Konstruktionen, Freie Verlagsanstalt, Stuttgart, 1982). Es hat nicht an Versuchen gefehlt, auch in der Chirurgie des Bewegungsapparates diese Prinzipien zu übernehmen, doch blieben diese Versuche ausschließlich auf die Osteosynthese beschränkt, d.h. auf das Verschrauben von Knochenbrüchen (Müller, Allgöwer und Willenegger, Manual der Osteosynthese, Springer Verlag, Heidelberg, Berlin, New York, 1969).

Im Bereich der Prothetik wurden bereits Konstruktionen beschrieben, die aus Metall oder Kohlenstoff bestehen und eine Armierung des Knochenzements bewirken, beispielsweise Netzwerke um Prothesenschäfte, wie sie in der US-A-4 064 567 beschrieben sind. Diese Netzwerke bilden keinen geschlossenen Köcher und sind nicht flexibel. Aufgrund ihrer Konstruktion können sie selbst keine Verbindung zum knöchernen Lager der Prothese aufnehmen und stellen somit selbst in sich als Gesamtkonstruktion eine Prothesenkomponente dar.

In der DE-A-29 17 446 (=EP-A-0 018496) werden mehrlagige strumpfähnliche Armierungen in Form von chirurgischen Netzwerken beschrieben, die teilweise resorbierbar sind und eine definierte Maschenweite und Fadendicke aufweisen. Diese Armierungen sind zu einer exzellenten Verstärkung des Zementköchers einer Prothese geeignet, sie sind jedoch kaum ausreichend in der Lage, die ungünstigen Scherkräfte abzufangen, die im Interface der Prothesenkomponenten zum Knochen auftreten. Außerdem bekommen die reinen Drahtarmierungen gemäß der DE-A-29 17 446 aufgrund ihrer geringen Affinität zum PMMA des Knochenzements keinen idealen Kontakt zur Zementmatrix und können Fülleffekte erzeugen, die wiederum Sollbruchstellen darstellen. Es kann deshalb bei hoher Beanspruchung zum Wandern der Armierung und zur Zerstörung der Matrix kommen. Die resorbierbaren Materialien gemäß der DE-A-29 17 446 fungieren als Platzhalter für den Knochen; sie sind allein jedoch möglicherweise nicht in der Lage, auf Dauer eine Kraftübertragung von der Prothesenkomponente auf den Knochen zu ermöglichen.

EP-A-0 159 036 beschreibt eine Beschichtungsmasse und ein Verankerungsteil für Implantate mit ganz oder teilweise beschichteter Oberfläche. In einer speziellen Ausführungsform kann die Beschichtungsmasse eine zusätzliche Armierung aufweisen, die als geschlossenes Netz in Form eines Prothesenstrumpfes ausgebildet sein kann. Der Prothesenstrumpf kann aus Kugelketten bestehen. Das Netz kann auch als mehrschichtiger Strumpf ausgebildet sein. Der Strumpf wird zunächst über die Prothese gestülpt, vorzugsweise in mehreren Schichten, und kann anschließend unter beliebiger Vorspannung auf dem Prothesenschaft gehalten werden. Der mit dem Strumpf überzogene Prothesenschaft wird mit der Beschichtungsmasse beschichtet, die anschließend getrocknet wird und einen geschlossenen Mantel um das Verankerungsteil der Prothese ausbildet. In diesem Zustand wird das Verankerungsteil in die Markhöhle implantiert.

Aus der EP-A-0 006 414 ist es bekannt, Knochenzement zur Erhöhung der mechanischen Festigkeit und der Körperverträglichkeit mit einem Kohlefaser-Strumpf zu verstärken und mit Apatit zu vermischen. Der Kohlefaser-Strumpf kann in einer speziellen Ausführungsform als geschlossener Strumpf hergestellt werden. Um den Knochenzement mit dem Kohlefaser-Strumpf in die Markhöhle des Knochens einzubringen, wird der Strumpf mit Knochenzement ausgegossen und im feuchten Zustand in die Markhöhle gebracht, und anschließend wird der Prothesenschaft hineingetrieben.

Die Prothesenstrümpfe gemäß EP-A-0 159 036 und EP-A-0 006 414 sind somit jeweils als Armierung fest in die Prothesenbeschichtung bzw. in den Knochenzement eingebracht.

Ferner ist aus Langzeitstudien bekannt, daß der herkömmliche Knochenzement ausgezeichnet und mehr als 20 Jahre im Körper auf Druck beansprucht werden kann, daß er aber gegenüber Zug- und Biegebeanspruchung keine ausreichende Resistenz aufweist und innerhalb kurzer Zeit der Beanspruchung erliegen kann.

Der Erfindung liegt somit die Aufgabe zugrunde, eine Vorrichtung gemäß dem Oberbegriff des Anspruchs 1 bereitzustellen, mit der die Zug- und Biegekräfte auf das knöcherne Lager und den Knochenzement in Druckkräfte umgewandelt werden können.

Diese Aufgabe wird mit der vorliegenden Erfindung gemäß dem Patentanspruch 1 gelöst. Erfindungsgemäß wird der Knochenzement so verstärkt bzw. das Implantat so im knöchernen Lager aufgehängt, daß lediglich Druckkräfte auftreten. Die Implantation des erfindungsgemäßen chirurgischen Trag- und Netzwerkes führt zu einer Ausschaltung der schädlichen Scherkräfte und damit zur erheblichen Verlängerung der Implantationszeit.

Gegenstand der Erfindung ist somit ein chirurgisches Trag- und Netzwerk, welches so im Knochen verspannbar bzw. Vorspannbar ist, daß die auf die verankerte Prothesenkomponente einwirkenden Kräfte gleichmäßig auf das Knochenlager übertragen werden.

Das erfindungsgemäße Trag- oder Netzwerk ist köcherförmig ausgebildet und besteht aus mindestens einem zu dem Netzwerk verstrickten Faden. Der Faden selbst ist vorzugsweise flexibel; in jedem Falle sollte aber das aus dem Faden gestrickte köcherförmige Netzwerk in seiner Längsrichtung flexibel sein, so daß es bei seiner Verspannung im Knochen vorzugsweise um mindestens 5%, besonders bevorzugt um etwa 10 bis 20% seiner Gesamtlänge im unverspannten Zustand gedehnt werden kann. Der Köcher des Trag- oder Netzwerks kann an seinem unteren Ende offen oder geschlossen sein.

Das köcherförmige Netzwerk kann einlagig sein. Die mehrlagige Ausführungsform, die beispielsweise durch ein- oder mehrmaliges Umstülpen des auf einen Zylinder aufgezogenen Strumpfes erhalten wird, wie es in der DE-A-29 17 446 beschrieben ist, weist jedoch erhöhte Stabilität und Festigkeit auf. Die Ausführung kann im einzelnen den biomechanischen Anforderungen angepaßt werden. Die folgende Beschreibung erfolgt in erster Linie am Beispiel einer Hüftgelenksprothese, das erfindungsgemäße Trag- und Netzwerk ist jedoch für alle Gelenkersatzkomponenten anwendbar, beispielsweise am Knie, Sprung- oder Ellbogengelenk.

Das erfindungsgemäße Trag- oder Netzwerk ist so ausgebildet, daß es vorspannbar ist, so daß es beim Aufhängen im knöchernen Lager oder beim Einbringen des Knochenzements in das knöcherne Lager gedehnt wird und in diesem gedehnten Zustand verbleibt.

Zum Erzeugen der Vorspannung sind verschiedene Ausführungsformen vorgesehen. Beispielsweise kann ein Ende des Köchers mit einem sich verblockenden Kunststoff beispielsweise in der Markhöhle des proximalen Femurs verblockt werden und das andere Ende kann von Hand oder mit einem Spanngerät über die Metaphyse gezogen und dort mit Schrauben, Stiften, Dübeln oder speziellen Knochendübeln, wie sie beispielsweise in der DE-A-34 45 738 beschrieben sind, unter Spannung fixiert werden.

Bei einer weiteren Ausführungsform kann der Faden, aus dem das Netzwerk besteht, die Form einer Kugelkette aufweisen, d.h. der Faden kann beispielsweise viele aufeinanderfolgende kugelförmige Verdickungen aufweisen, die sich beim Extrudieren erzeugen lassen, oder die Kugeln können auf den Faden aufgepreßt werden, beispielsweise im HIP-Verfahren (High Isostatic Pressure-Verfahren). Diese Kugeln weisen vorzugsweise einen Durchmesser von etwa 0,5 mm bis 3 mm auf, vorzugsweise etwa 1 mm bis 2 mm, und einen Abstand von vorzugsweise etwa 0,5 bis 2 cm voneinander. Kugeln dieses Durchmessers können auch als Platzhalter für die entsprechende Zementschichtdicke dienen. Wenn das Netzwerk aus Kugelketten dieses Durchmessers aufgebaut ist, bauen sich beim Einbringen des Knochenzements an den Kugeln hohe Widerstände auf, die zu einer Dehnung (Elongierung) und damit Vorspannung des Köchers führen. In Abhängigkeit von der Viskosität des Zements können hierzu auch gegebenenfalls Kugeln kleineren Durchmessers geeignet sein, der Durchmesser sollte jedoch in jedem Fall mehr als 200 µm betragen. Bei kleineren Kugeldurchmessern kommt es zu keiner ausreichenden Vorspannung des Netzwerks.

Ferner ist es auch möglich, Teile des köcherförmigen Netzwerks seitlich aus dem Köcher herauszuführen, und die seitlichen Fortsätze oder Vorsprünge des Netzwerks durch schräge Bohrungen in verschiedenen Etagen des Prothesenschaftes und gegebenenfalls auch unterhalb dessen Spitze nach außen zu führen und dort dreidimensional zu verspannen, beispielsweise mit Hilfe von Schrauben oder Nähten.

Eine Vorspannung läßt sich auch dadurch erzielen, daß vom Innern des Köchers her ein Druck auf das Trag- oder Netzwerk ausgeübt und dieses dadurch gedehnt wird. Dies kann beispielsweise mittels eines in den Köcher eingebrachten und geeignet geformten aufblasbaren Ballons erfolgen.

Durch die vorstehend beschriebene Vorspannung des Netzwerkes gelingt es, eine sehr gleichmäßige Kraftübertragung auf den Knochen zu erzielen.

Besonders vorteilhaft ist es, für das Netzwerk zur Verstärkung des Knochenzementes "isogene" Materialien zu verwenden, vorzugsweise Materialien, die die gleiche chemische Herkunft wie der Knochenzement bzw. chemische Affinität mit dem Knochenzement aufweisen und/oder in der Lage sind, eine chemische Verbindung mit dem flüssigen Knochenzement einzugehen. Da alle derzeit in der Chirurgie des Bewegungsapparates eingesetzten Knochenzemente Polymethylmetacrylate sind, die kalt polymerisieren, sind "isogene" Materialien in erster Linie Acrylatfasern oder mit Acrylaten beschichtete Metallfäden und Drähte. Die Fäden des erfindungsgemäßen Netzwerks bestehen deshalb vorzugsweise aus einem Acrylat, Metacrylat, PMMA und/oder dessen Copolymeren oder Derivaten und/oder aus einem zu den Acrylaten affinen Kunststoff.

Je nach Beanspruchung der Gesamtkonstruktion können auch armierte Acrylatfäden besonders vorteilhaft sein, beispielsweise mit Acrylat beschichtete Metallfäden. Die Metallfäden können sowohl monofil als auch bei besonders hoher Beanspruchung polyfil sein. Als Metall ist insbesondere Titan geeignet oder ein chirurgischer Stahldraht mit gewisser Elastizität, beispielsweise der sterile Metalldraht EH7602 der Fa. Ethicon. Wenn die sekundär polymerisierende Matrix des Knochenzementes mit einem solchen Netzwerk eine chemische Verbindung eingeht, ist eine um ein vielfaches höhere und sicherere Kraftübertragung von der Prothesenkomponente auf den Zement und vom Zement auf den Knochen, aber auch direkt von der Armierung auf den Knochen möglich. Beim letzteren ist es von ausgesprochenem Vorteil, wenn die Ummantelung des Metalles wiederum zu einer Dämpfung der direkten Krafteinleitung auf den Knochen beiträgt. Es ist im Rahmen der Erfindung aber auch möglich, Metallfäden, beispielsweise aus Titan oder einem chirurgischen Stahldraht, als Material für das Netzwerk zu verwenden.

Vorzugsweise ist das Material des Netzwerks "isoelastisch", d.h. es weist etwa die gleiche Elastizitätskonstante wie der Knochenzement auf, um eine besonders gute Kraftübertragung zu ermöglichen.

Die Fadendicke hängt vom verwendeten Material ab und beträgt vorzugsweise etwa 100 bis 700 µm. Bei Verwendung von Acrylat beträgt die Fadendicke vorzugsweise etwa 100 bis 300 µm, besonders bevorzugt etwa 200 µm, bei Verwendung von Titan vorzugsweise etwa 200 bis 500 µm, besonders bevorzugt etwa 400µm, und bei Verwendung von chirurgischem Stahl mehr als 50 µm, vorzugsweise etwa 100 bis 300 µm, besonders bevorzugt etwa 200 µm. Die Maschenbreite bzw. -dicke beträgt 0,5 bis 10 mm, vorzugsweise etwa 1 bis 4 mm, besonders bevorzugt etwa 1,5 mm.

Durch den teilweisen Einbau von resorbierbaren Netzen kann durch die Resorption dieser Strukturen und den nachfolgenden Knocheneinwuchs die kraftaufnehmende Fläche des Knochens noch vergrößert werden. Als resorbierbare Materialien kommen insbesondere organische Polymere, wie Catgut oder ein anderes Kollagen, ein synthetisches Polylactid, Polyglycolid oder ein anderes Polyaminosäurederivat oder verwandtes Polymer und/oder ein Gemisch verschiedener organischer Polymere in Betracht. Vorzugsweise werden die resorbierbaren Materialien im proximalen Teil und/oder in der äußersten Lage des Netzwerks eingesetzt. Falls die Fäden mit Kugeln bestückt sind oder als Kugelketten ausgebildet sind, werden besonders vorteilhaft gesinterte Apatitkugeln oder Kugeln aus einem Verbund mit Tricalciumphosphat verwendet. Dies hat einerseits den Vorteil, daß die Kugeln als formgebende Elemente für die Bildung einer physiologischen Knochenwabe wirken und Apatite aufgrund ihrer hohen Affinität zur Knochenzelle besonders günstige Materialeigenschaften aufweisen (vgl. K. Draenert: Some New Observations to Improve the Bone-To-Cement Contact, Nicholas Andry Award Paper, presented at the 38th annual Meeting of The Association of Bone and Joint Surgeons, held in Vancouver, May 27-31, 1986). Andererseits geht von gesinterten harten Füllern auch eine positive biomechanische Induktion der Knochenbildung aus, wie in Tierversuchen bewiesen werden konnte.

Vorteilhafterweise werden die einzelnen Lagen des Netzwerks in Radialrichtung des Köchers durch mindestens einen Distanzhalter, beispielsweise einen Spannring, in definiertem Abstand voneinander gehalten. Der Abstand beträgt etwa 0.5 - 5 mm, vorzugsweise etwa 1 mm.

Mit dem erfindungsgemäßen Trag- und Netzwerk steht dem operativ tätigen Orthopäden und Chirurgen ein Material zur Verfügung, das das Problem der Beanspruchung des Zements bzw. bei zementfreien Implantaten das Problem des Auftretens von Spannungsspitzen (Streßkonzentration) in vorteilhafter Weise löst.

Die Erfindung wird nachstehend anhand von Beispielen und anhand der Zeichnungen näher erläutert. Es zeigen:
Fig. 1 ein erfindungsgemäßes Netzwerk in Form eines zweilagigen Strumpfes, und
Fig. 2 eine erfindungsgemäße, netzförmige Pfannenverstärkung.

### Beispiel 1

Zur Herstellung eines Trag- und Netzwerkes für die Aufnahme einer femoralen Gelenkersatzkomponente des Hüftgelenks werden 100 m eines chirurgischen Titandrahtes mit einer Fadendicke von 400 µm auf einer handelsüblichen Rundstrickmaschine zu einem endlosen Strumpf verstrickt. Es wird eine Maschenweite von etwa 1 bis 2 mm eingestellt und ein Umfang des Strumpfes weist 14 Maschen auf. Durch Aufziehen des Strumpfes über einen Spannring erhält man eine zweilagigen Strumpf, der mittels eines zweiten Spannringes am proximalen Ende konstante Lagenabstände aufweist und der durch Verschrauben des proximalen Ringes durch hierfür vorgesehene Schraubenlöcher im Knochen und durch Spannen des distalen Ringes durch Verblocken in der Markhöhle mit einer einstellbaren Vorspannung im knöchernen Lager verankert wird.

### Beispiel 2

100 m eines 400 µm dicken Titandrahtes werden im Tauchverfahren mit einem Acrylat beschichtet und nach dem Trocknen aufgerollt und zur weiteren Verarbeitung in einer Rundstrickmaschine vorbereitet. 50 m des so beschichteten Drahtes werden auf die Spulen der Rundstrickmaschine aufgerollt und zu einem Netzwerk verstrickt, dessen Maschen eine Größe von etwa 1 mm aufweisen, wobei das strumpfförmige Netzwerk entlang seines Umfanges 40 Maschen aufweist. Das Netzwerk wird über einen Spannring aufgerollt und das freie Ende des Rundgestrickes wird ebenfalls über einen verspannbaren Ring oder über einen Knochendübel befestigt. Der Ring wird mit Schraubenlöchern zur Aufnahme von Knochenschrauben versehen.

### Beispiel 3

Auf einen 400 µm dicken Titandraht werden im HIP-Verfahren in Abständen von jeweils 2 cm Kugeln aus Tricalciumphosphat mit einem Durchmesser von 1 mm aufgepreßt. Die so entstandene Kugelkette wird im oben beschriebenen Verfahren auf die Spulen einer Rundstrickmaschine aufgezogen, wobei die Nadeln der Maschine in diesem Fall nicht an die Drahtdicke, sondern an die Kugeln angepaßt werden müssen. Die weitere Verarbeitung erfolgt wie beschrieben.

### Anwendungsbeispiel 1

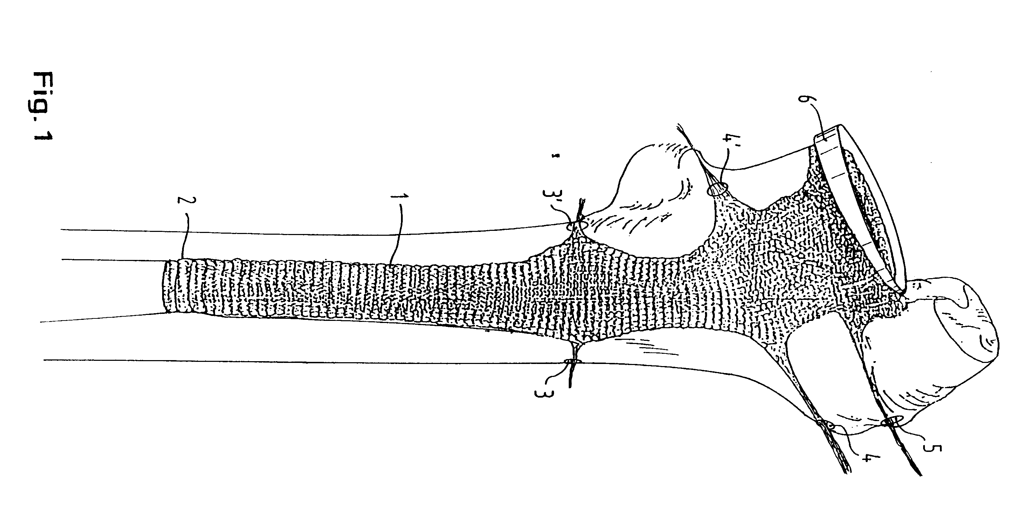

Figur 1 zeigt einen zweilagigen Strumpf 1, der an seiner Spitze mit einem verblockbaren Knochendübel 2 in der Markhöhle fixiert und über in drei Etagen übereinander angeordnete Bohrlöcher 3,3',4,4',5 nach außen gezogen, fixiert und vorgespannt ist. Das proximale Ende wird nach dem Spannen mit einem Spanninstrument mit einer Titankappe 6 über dem Schenkelhals fixiert. Die Kappe kann auch als Distanzhalter zwischen den Lagen des Netzwerks dienen.

### Anwendungsbeispiel 2

Ein arthrotisch verändertes Hüftgelenk wird zur Aufnahme eines Gelenkersatzes vorbereitet. Nach der präoperativen Planung wird der Hüftkopf in entsprechender Höhe reseziert und die Femurmarkhöhle mit einer Diamanthohlfräse lateral eröffnet. Vorteilhafterweise kann die Eröffnung der Markhöhle der Resektion von Kopf und Hals des Femurs vorangehen. Zur Vorbereitung des Knochenbetts gehört die sorgfältige Drucklavage der Markhöhle. Danach wird mit Hilfe eines Troikars distal der Prothesenspitze mit Hilfe eines 4,5 mm dicken Bohrers, einer Bohrführung und einer Druckluftbohrmaschine eine Drainageöffnung an der anterolateralen Zirkumferenz des Femurs gesetzt. In dieses Bohrloch wird durch die Bohrführung eine selbstschneidende Kanüle eingesetzt. Das in Beispiel 1 hergestellte chirurglsche Netzwerk wird daraufhin mit einem gewaschenen Spongiosazylinder in die Markhohle des Femurs eingeführt und mit einem Stößel distal in der Markhöhle verblockt. Mit Hilfe eines Spanngerätes wird nun der proximale Ring unter Vorspannung (Elongation) des Netzwerks auf die proximale Femuröffnung aufgesetzt und verschraubt. Danach wird die Markhöhle durch Aufsetzen einer Zementspritze, wie sie beispielsweise in der EPA-85 10 8607 beschrieben ist, mit einer Gummidichtung hermetisch verschlossen und durch Anlegen eines Vakuums an der distalen Drainageöffnung und gleichzeitiges Auspressen der Zementspritze wird Knochenzement durch das Netzwerk bis in den Knochen eingefüllt. Danach wird die Prothesenkomponente eingeführt und nicht mehr berührt, bis die Aushärtung des Knochenzements abgeschlossen ist.

### Anwendungsbeispiel 3

Ein einfaches und wirksames Netzwerk zur Verstärkung des Knochenzements wird in der Weise hergestellt, daß 100 m einer 200 µm dicken Acrylatfaser, beispielsweise einer handelsüblichen Lichtleiterfaser, auf einer Rundstrickmaschine zu einem Rundgestrick verstrickt werden, das eine Maschenweite von etwa 1,5 mm und jeweils 50 Maschen entlang seines Umfangs aufweist. Ein solches Acrylatfasernetz wird über einen Spannring mit 44 mm Durchmesser aufgerollt, und das freie Ende des Netzes wird nach vier Lagen über einem Ring mit 16 mm Durchmesser gedoppelt. Dieses Netzwerk kann bei dem im Anwendungsbeispiel 2 beschriebenen Fall einer schweren Arthrose eingesetzt werden. Dabei wird die Pfanne des erkrankten Hüftgelenks von dem noch verbliebenen Knorpel befreit und mit Hilfe einer Diamanthohlschleife mit 16 mm Durchmesser, wie sie beispielsweise in der DE-OS 32 02 193 beschrieben ist, wird ein Defekt im tragenden Pfannendach gesetzt. Der Köcher des Trag- und Netzwerkes wird daraufhin mit einem verschraubbaren Dübel in der Richtung auf das Sacroiliacalgelenk, d.h. das Gelenk, über welches die Kraft auf die Wirbelsäule übertragen wird, im Becken fixiert. Danach wird das Netzwerk mit Hilfe eines Spanngerätes vorgespannt und mittels des Spannringes am Azetabularrand verschraubt. Nach sorgfältiger Lavage des knöchernen Lagers erfolgt das Einbringen einer Drainageschraube vom lateralen Pfannenrand aus und das Auffüllen mit Knochenzement unter dem Sog des über die Drainageschraube angelegten Vakuums. In den so vorbereiteten und eingebrachten Knochenzement wird die Pfanne vorsichtig eingedrückt.

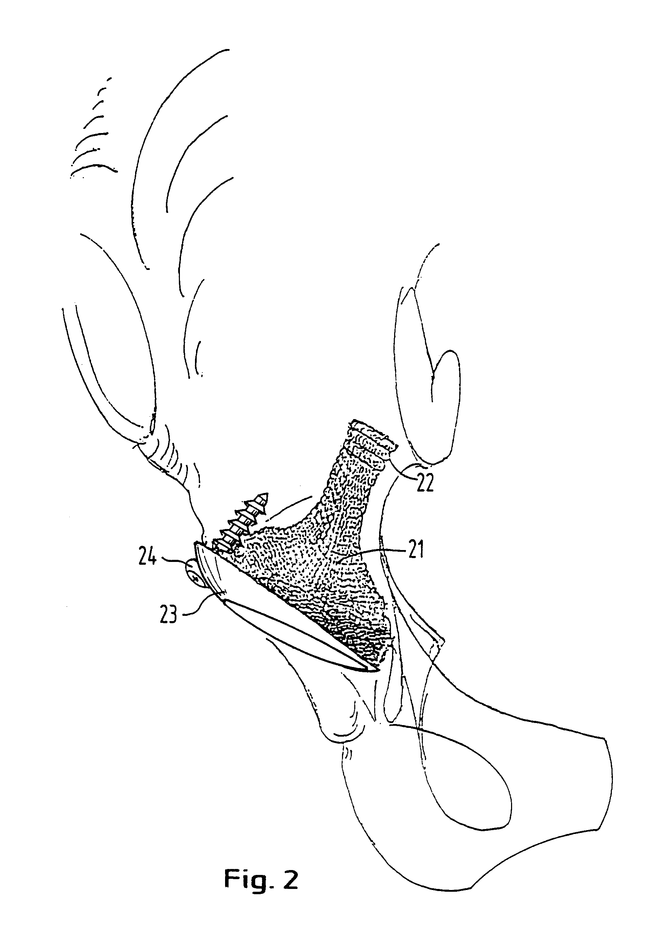

In Fig. 2 ist eine derartige Pfannennetzverstärkung 21 dargestellt. Durch ein Schleifloch mit 16 mm Durchmesser wird das Ende des Köchers mit Hilfe eines Knochendübels 22 im Becken fixiert. Im Anschluß daran wird das Netzwerk mit einem Spanngerät vorgespannt und mit einem Titanring 23 mittels einer Knochenschraube 24 am Beckenrand fixiert. Nunmehr ist das Lager vorbereitet für die Aufnahme von Knochenzement und Pfanne.

## Patentansprüche

1. Chirurgisches Trag- oder Netzwerk zur Verankerung einer Endoprothese und/oder zur Verstärkung des zur Verankerung einer Endoprothese verwendeten Knochenzements, mit einem Prothesenköcher (1; 21) aus ein- oder mehrlagig angeordneten Fäden zur Aufnahme der Endoprothese, gekennzeichnet durch Mittel zum Verankern eines Endes des Prothesenköchers (1; 21) im Knochenlager, zum Vorspannen des Prothesenköchers im Knochenlager und zum Fixieren des vorgespannten Prothesenköchers im Knochenlager.

2. Trag- oder Netzwerk nach Anspruch 1, dadurch gekennzeichnet, daß die Fäden aus Metall und/oder aus Polymeren bestehen und/oder mit Polymeren beschichetete Metallfäden sind.

3. Trag- oder Netzwerk nach Anspruch 1 oder 2, dadurch gekennzeichnet, daß als Material für die Fäden ein mit einem Acrylat, Metacrylat, Polymetacrylat und/oder mit einem verwandten Derivat der Acrylate beschichteter mono- oder polyfiler Draht aus Titan oder einem anderen biokompatiblen Material verwendet wird.

4. Trag- oder Netzwerk nach Anspruch 1 oder 2, dadurch gekennzeichnet, daß als Material für die Fäden ein Acrylat, Metacrylat, Polymethylmetacrylat und/oder deren Copolymere oder Derivate und/oder ein zu den Acrylaten chemisch affiner Kunststoff verwendet wird.

5. Trag- oder Netzwerk nach einem der Ansprüche 1 bis 4, dadurch gekennzeichnet, daß es zumindest teilweise resorbierbar ist, wobei als resorbierbares Material ein organisches Polymer, wie Catgut oder ein anderes Kollagen, ein synthetisches Polylactid, Polyglycolid oder ein anderes Polyaminosäurederivat oder verwandtes Polymer und/oder ein Gemisch verschiedener organischer Polymere verwendet wird.

6. Trag- oder Netzwerk nach einem der Ansprüche 1 bis 5, dadurch gekennzeichnet, daß als Material für die Fäden Kugelketten verwendet werden, deren Kugeln einen Durchmesser von etwa 500 bis 3000 µm, vorzugsweise etwa 1000 bis 2000 µm aufweisen.

7. Trag- oder Netzwerk nach Anspruch 6, dadurch gekennzeichnet, daß die Kugelketten aus Keramik, Apatit oder einem anderen Tricalciumphosphatderivat oder Calciumphosphat in gesinterter oder ungesinterter Form und/oder aus einem biokompatiblen Metall oder beschichteten Metall bestehen.

8. Trag- oder Netzwerk nach einem der Ansprüche 1 bis 7, dadurch gekennzeichnet, daß der Protherenköcher (1; 21) etagenweise und/oder an einem seiner beiden Enden unter Verwendung von Schrauben, Klammern, Knochendübeln (2), Stiften und/oder Nähten vorspannbar ist, oder daß er durch Innendruck vorspannbar ist.

9. Trag- oder Netzwerk nach Anspruch 8, gekennzeichnet durch mindestens einen selbstverblockenden oder verblockbaren Dübel aus einem biokompatiblen Kunststoff oder Metall und/oder ein Knochentransplantat zum Verspannen des Prothesenköchers.

10. Trag- oder Netzwerk nach einem der Ansprüche 1 bis 9, gekennzeichnet durch Distanzhalter, die die einzelnen Lagen der Fäden etagenweise und/oder an einem der beiden Enden des Protherenköchers in einem definierten Abstand von 0,5 bis 5 mm, vorzugsweise 1 mm voneinander halten.

## Claims

1. A surgical support or network for anchoring an endoprosthesis and/or reinforcing the bone cement used for anchoring an endoprosthesis, comprising a prosthesis quiver (1; 21) made of one or more layers of threads for receiving the endoprosthesis, characterized by means for anchoring one end of the prosthesis quiver (1; 21) in the bony bed, for prestressing the prosthesis quiver in the bony bed and for fastening the prestressed prosthesis quiver in the bony bed.

2. The support or network according to claim 1, characterized in that the threads are made of metal and/or polymers and/or are polymer-coated metal threads.

3. The support or network according to claim 1 or 2, characterized in that the material for the threads is monofilic or polyfilic wire made of titanium or another biocompatible material and being coated with an acrylate, methacrylate, polymethacrylate and/or with a related derivative of the acrylates.

4. The support or network according to claim 1 or 2, characterized in that the material for the threads is an acrylate, methacrylate, polymethyl methacrylate and/or the copolymers or derivatives thereof and/or a synthetic material which is chemically affinitive to the acrylates.

5. The support or network according to any one of claims 1 to 4, characterized in that the support or network is at least partially absorbable, the absorbable material being an organic polymer, such as catgut or another collagen, a synthetic polylactide, polyglycolide or another polyamino acid derivative or related polymer and/or a mixture of various organic polymers.

6. The support or network according to any one of claims 1 to 5, characterized in that bead chains whose beads have a diameter of about 500 to 3000 µm, preferably about 1000 to 2000 µm, are used as the material for the threads.

7. The support or network according to claim 6, characterized in that the bead chains are made of ceramics, apatite or another tricalcium phosphate derivative or calcium phosphate in sintered or non-sintered form and/or of a biocompatible metal or coated metal.

8. The support or network according to any one of claims 1 to 7, characterized in that the prosthesis quiver (1; 21) is prestressable in tiers and/or at one of its two ends using screws, clamps, bone dowels (2), pins and/or seams, or in that the quiver is prestressable by means of internal pressure.

9. The support or network according to claim 8, characterized by at least one self-locking or interlockable dowel made of a biocompatible plastic material or metal and/or a bone transplant for prestressing the prosthesis quiver.

10. The support or network according to any one of claims 1 to 9, characterized by spacers which separate the individual layers of the threads in tiers and/or at one of the two ends of the prosthesis quiver at a predetermined distance of 0.5 to 5 mm, preferably 1 mm.

## Revendications

1. Structure réticulée ou portante pour applications chirurgicales pour l'ancrage d'une prothèse terminale et/ou pour le renforcement du ciment osseux utilisé pour l'ancrage d une endoprothèse avec un étui de prothèse (1; 21) constitué de fils disposés en une ou plusieurs couches destinées à recevoir l'endoprothèse, caractérisée par des moyens pour ancrer une extrémité de l'étui de prothèse (1; 21) dans le support osseux pour précontraindre l'étui de prothèse dans le support osseux et pour fixer l'étui de prothèse précontraint dans le support osseux.

2. Structure réticulée ou portante selon la revendication 1, caractérisée en ce que les fils sont constitués de métal et/ou de polymères et/ou sont des fils métalliques enduits de polymère.

3. Structure réticulée ou portante selon la revendication 1 ou 2, caractérisée en ce que le matériau utilisé pour les fils est du fil de titane ou d'un autre matériau biocompatible de type mono ou polyfil, enduit d'un acrylate, d'un méthacrylate, d'un polyméthacrylate et/ou d'un dérivé apparenté des acrylates.

4. Structure réticulée ou portante selon la revendication 1 ou 2, caractérisée en ce que le matériau utilisé pour les fils est un acrylate, un méthacrylate, un polyméthacrylate de méthyle et/ou un de leurs copolymères ou dérivés et/ou une matière plastique ayant une affinité chimique pour les acrylates.

5. Structure réticulée ou portante, selon l'une des revendications 1 à 4, caractérisée en ce qu'elle est au moins partiellement résorbable, tandis que le matériau résorbable est un polymère organique tel que le catgut ou un autre collagène, un polylactide synthétique, un polyglycolide ou un autre dérivé d'un acide polyaminé ou un polymère apparenté et/ou un mélange de différents polymères organiques.

6. Structure réticulée ou portante selon l'une des revendications 1 à 5, caractérisée en ce que l'on utilise comme matériau pour les fils des chaînes de billes dont les billes ont un diamètre d'environ 500 à 3000 µm et, de préférence, d'environ 1000 à 2000 µm.

7. Structure réticulée ou portante selon la revendication 6, caractérisée en ce que les chaînes de billes sont constituées de céramique, d'apatite ou d'un autre dérivé du phosphate tricalcique ou de phosphate calcique sous forme de phosphate fritté ou non fritté et/ou d'un métal biocompatible ou d'un métal enduit.

8. Structure réticulée ou portante selon l'une des revendications 1 à 7, caractérisée en ce que l'étui de prothèse (1; 21) peut être précontraint par étages et/ou à l'une de ses extrémités en utilisant des vis, des pinces, des goujons pour os (2), des broches et/ou des coutures ou en ce qu'il peut être précontraint par une pression intérieure.

9. Structure réticulée ou portante, selon la revendication 8, caractérisée par au moins un goujon autobloquant ou blocage, en matière plastique ou métal biocompatible et/ou un transplant d'os pour précontraindre l'étui de prothèse.

10. Structure réticulée ou portante, selon l'une des revendications 1 à 9, caractérisée par des entretoises qui maintiennent, à une distance définie les unes des autres de 0,5 à 5 mm et, de préférence, de 1 mm, les différentes couches des filaments par étage et/ou à l'une des deux extrémités de l'étui de prothèse.
